# EUROPEAN PATENT APPLICATION

(11) **EP 4 231 013 A2**
(43) Date of publication of application: **23.08.2023**
(21) Application number: 22209503.6
(22) Date of filing: 24.11.2022
(51) Int. Cl.: G01N 33/493, G01N 33/487, G07F 17/04

(54) **KIOSK TYPE URINE ANALYZER AND HEALTH MONITORING SYSTEM USING THE SAME**

(30) Priority: 16.02.2022 KR 20220020436
(71) Applicant: Lee, Myung Oh, Gwangju 62262 (KR)
(72) Inventor: Lee, Myung Oh, Gwangju 62262 (KR)
(74) Representative: Stolmár & Partner Patentanwälte PartG mbB

(57) **Abstract**

The present invention relates to a kiosk type urine analyzer and a health monitoring system using the same and relates to a kiosk type urine analyzer capable of analyzing urine of a user in an unmanned manner to provide an analysis result and monitoring health of the user through the analysis result, and a health monitoring system using the same.

## Description

### CROSS-REFERENCE TO RELATED APPLICATION

This application claims priority to and the benefit of Korean Patent Application No. 10-2022-0020436, filed on February 16, 2022, the disclosure of which is incorporated herein by reference in its entirety.

### BACKGROUND

### 1. Field of the Invention

The present invention relates to a kiosk type urine analyzer and a health monitoring system using the same and relates to a kiosk type urine analyzer capable of analyzing urine of a user in an unmanned manner to provide an analysis result and monitoring health of the user through the analysis result, and a health monitoring system using the same.

### 2. Discussion of Related Art

In general, among methods of checking health, the most widely used methods are a blood test and a urine test. A blood test is widely used as a method of diagnosing diseases in the body based on the fact that waste products and water which are components contained in urine are filtered through the kidneys and are excreted from the body. Conventional urine testers are manufactured for use in tests for patient treatment in hospitals, and therefore are expensive and also difficult to operate due to the specificity of a medical device and cannot be easily used by the general public. On the other hand, portable or home urine test devices have also been proposed to be used at home, but since portable or home urine test devices are also expensive, it is difficult for general users to easily access portable or home urine test devices.

In order to solve these difficulties, urine analysis vending machines that can be disposed in public places and used so that general users can easily use the urine analysis vending machines at low costs have been proposed. For example, Korean Patent Publication No. 10-2005-0091180 discloses a vending-machine urine analyzer which may be disposed in a public place such as a restroom and in which, when a test subject pays by inputting cash such as banknotes, a sample stick for a urine test is discharged, and when the test subject attaches urine to the discharged sample stick and inserts the sample stick, the urine is analyzed to print and output an analysis result on paper.

Meanwhile, Korean Utility Model Registration No. 20-0436314 discloses a vending-machine urine analyzer which is placed in a public place such as a restroom so that general users may purchase urine test strips and in which, when a user inserts the urine test strip dipped in urine, the urine test strip is detected to display the urine test strip on a manual display so that the user may directly check a color change of a urine test strip reaction portion with the naked eye, or the urine test strip is automatically checked to display any one of three stages of "normal/management/specialist consultation stages" on an automatic display unit.

The urine analysis devices provide an advantage in that a user can easily obtain urine analysis information at a low cost without needing to purchase an expensive home appliance and without visiting a hospital.

However, the conventional urine analysis devices have adopted only a simple analysis method of detecting only an input urine sample without user information related to the urine sample and performing determination according to a preset determination algorithm, the result analysis content of which is very simple, and thus there has been a limitation in that a urine test for a meaningful medical diagnosis is insufficient.

Korean Patent Publication No. 10-2349034 discloses a user-customized mobile urine analyzing method and system based on a urine analyzer vending machine in which urine analysis information is generated using a urine sample and additional information such as body information transmitted from a mobile user terminal, thereby rapidly and safely obtaining more accurate and detailed user-customized mobile urine analysis information.

The urine analysis vending machine may provide accurate and detailed user-customized urine analysis information, but there is a limitation in monitoring a health status of a user. The urine analysis vending machine has a structure in which a user takes out an analyzed strip again to discard the strip, and thus there is a disadvantage in that it is not hygienic.

Korean Patent Publication No. 10-2349034: User-customized mobile urine analyzing method and system based on urine analyzer vending machine.

### SUMMARY OF THE INVENTION

The present invention is directed to providing a kiosk type urine analyzer capable of providing a strip for testing urine of a user, providing monitoring target data that is a result of analyzing the urine of the user on the collected strip, and automatically collecting the strip on which urine analysis is completed.

The present invention is also directed to providing a health monitoring system using a kiosk type urine analyzer in which a data set is generated from data acquired by analyzing urine of a patient with a chronic disease and data acquired by analyzing urine of ordinary people without a chronic disease, and monitoring target data acquired by a kiosk type urine analyzer is input to a deep learning model constructed by learning the generated data set, thereby monitoring health of a user.

According to an aspect of the present invention, there is provided a kiosk type urine analyzer including a body which has an inner space and a strip outlet and a strip inlet which are formed to communicate with the inner space, a strip vending unit installed in the inner space and configured to store a plurality of strips for testing urine and provide the strips to users through the strip outlet, a strip accommodation unit configured to accommodate the strip with the urine which enters the inner space through the strip inlet, a urine analysis module configured to scan the strip put in the strip accommodation unit to analyze the urine, a touch screen through which unique identification information of the user is input or a request for the strip vending unit to discharge the strip to the strip outlet is input, a urine analysis control unit configured to control monitoring target data, which is acquired by the urine analysis module analyzing the urine on the strip, to be transmitted to an external device or displayed on the touch screen, and a local communication unit configured to transmit the monitoring target data to the external device.

The strip vending unit may include a strip accommodation chamber having an accommodating space which vertically extends therein for the plurality of strips to be stacked therein, an outlet formed in a lower portion of a side surface thereof facing the door to communicate with the accommodation space and the strip outlet, and a first roller entry hole formed to pass through a lower end portion thereof facing downward and communicate with the accommodation space, a withdrawal roller of which one side enters the first roller entry hole to support the strip disposed at a lowermost side among the plurality of vertically stacked strips, a strip pressing part configured to press the strip disposed at an uppermost side among the plurality of strips downward, and a withdrawal driving motor controlled and driven by the urine analysis control unit to rotate the withdrawal roller such that the strip supported by the withdrawal roller is discharged to the strip outlet.

The strip accommodation unit may include a strip holder having an accommodation hole of which one side communicates with the strip inlet to accommodate the strip, a light passing hole which passes through an upper end portion thereof to communicate with the accommodation hole such that light is radiated onto a plurality of reagent pads of the strip from the urine analysis module positioned above or light reflected from the reagent pads passes therethrough, and a second roller entry hole formed in a lower end portion thereof to communicate with the accommodation hole, an accommodation roller of which one side enters the second roller entry hole to support the strip which partially enters the accommodation hole, and an accommodation driving motor controlled and driven by the urine analysis control unit to primarily rotate the accommodation roller such that the strip supported by the accommodation roller is moved into the body in a direction away from the strip inlet.

The accommodation hole may be formed by opening the other end of the strip holder in a length direction, and the kiosk type urine analyzer may further include a first solenoid part including a first plunger which is movable to close the other end of the accommodation hole until the urine analysis module completes the analysis of the urine on the strip accommodated in the strip holder and open the other end of the accommodation hole when the analysis of the urine is completed, a waste strip collection chamber having a waste strip collection hole formed in an upper portion thereof such that, in a state in which the other end of the accommodation hole is opened, as the accommodation roller is secondarily rotated in the same direction as a direction in which the accommodation roller is primarily rotated, the strip discharged from the strip holder to the inner space of the body drops to be stored therein, a collection hole opening/closing door slidably mounted on an upper portion of the waste strip collection chamber to open or close the waste strip collection hole, and a second solenoid part including a second plunger which is movable together with the collection hole opening/closing door such that the waste strip collection hole is also opened or closed when the other end of the accommodation hole is opened or closed.

According to another aspect of the present invention, there is provided a health monitoring system including at least one kiosk type urine analyzer configured to provide a strip for urine test to a user and transmit monitoring target data acquired by analyzing urine on the collected strip to an external device, a database server configured to store each of patient group urine analysis data acquired by analyzing urine of a patient group requiring treatment for a chronic disease such as diabetes or nephritis, control group urine analysis data acquired by analyzing urine of a control group without a chronic disease, and past urine analysis data of the user, and a health monitoring server configured to, when the monitoring data transmitted from the kiosk type urine analyzer is input, monitor a health status of the user with respect to the chronic disease through a deep learning algorithm based on data stored in the database server and transmit a monitoring result to a user terminal configured to perform wireless communication with the kiosk type urine analyzer.

### BRIEF DESCRIPTION OF THE DRAWINGS

FIG 1 is a schematic block diagram of a health monitoring system using a kiosk type urine analyzer.
FIG 2 is a block diagram specifically illustrating a health monitoring server of FIG. 1.
FIG. 3 is a block diagram of a kiosk type urine analyzer according to a first embodiment of the present invention.
FIG. 4 is a perspective view of the kiosk type urine analyzer of FIG. 3.
FIG. 5 is a cross-sectional view of a portion of a strip vending unit of the urine analyzer of FIG. 3.
FIG. 6 is a cross-sectional view of a portion of a strip accommodation unit of the urine analyzer of FIG. 3.
FIG. 7 is an exploded perspective view of a strip kit of the urine analyzer of FIG. 3.
FIG. 8 is a cross-sectional view of a portion of a kiosk type urine analyzer according to a second embodiment of the present invention.

### DETAILED DESCRIPTION OF EXEMPLARY EMBODIMENTS

Hereinafter, a kiosk type urine analyzer and a health monitoring system using the same will be described in detail with reference to the accompanying drawings.

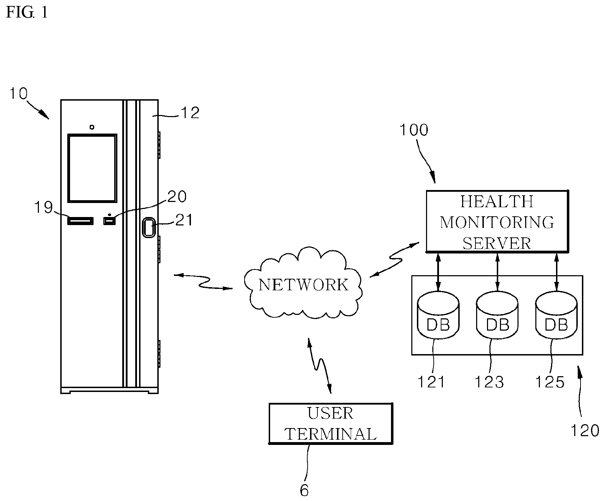

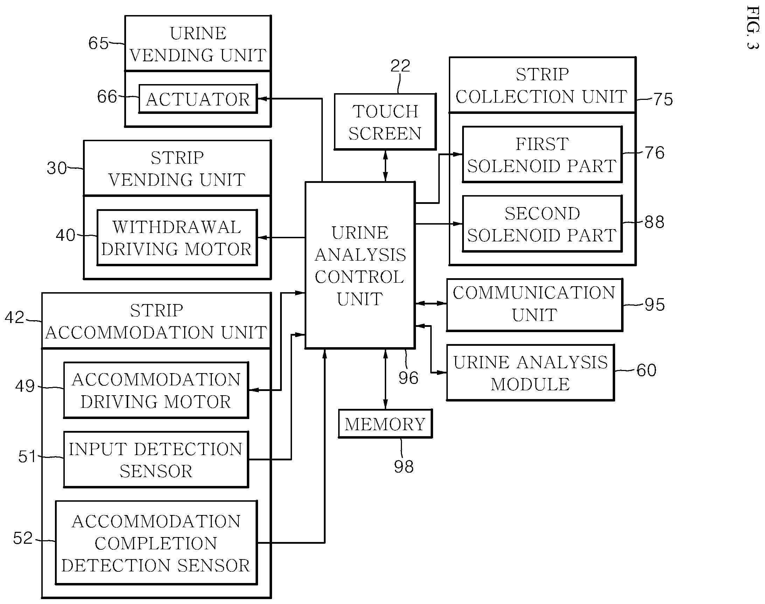

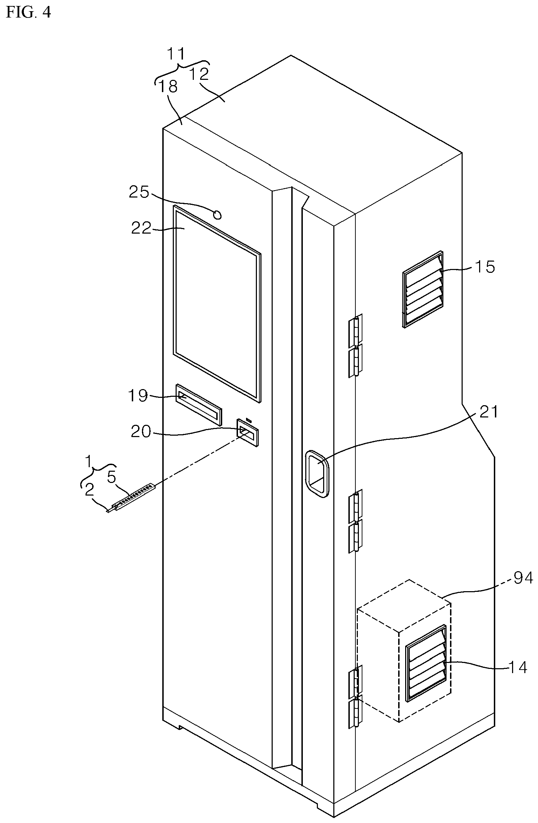

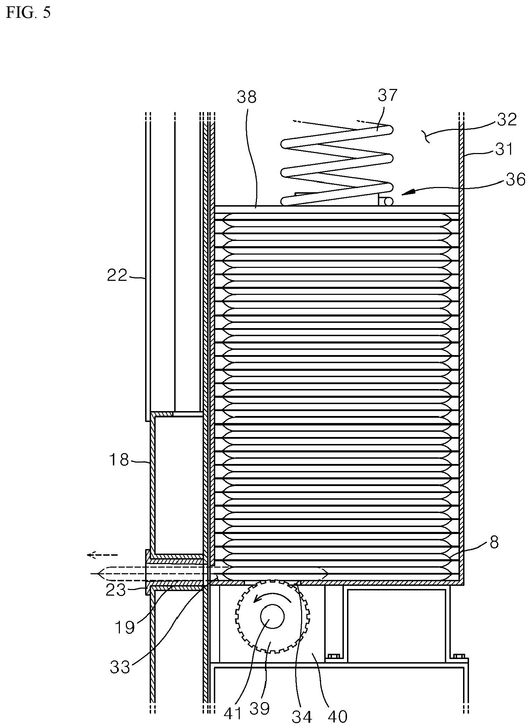

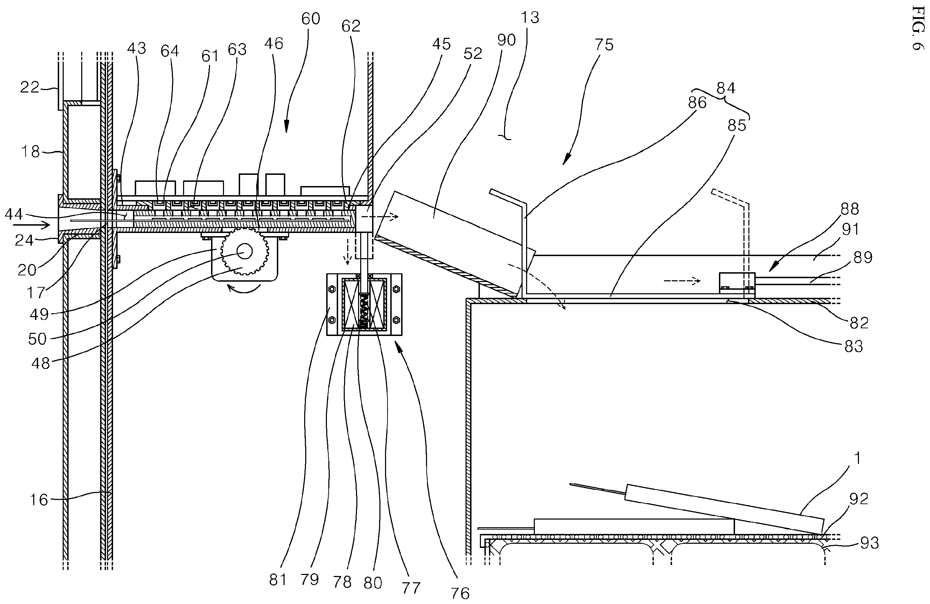

Referring to FIGS. 1 to 7, a kiosk type urine analyzer 10 according to a first embodiment of the present invention includes a body 11 having an inner space 13 and a strip outlet 19 and a strip inlet 20 which are formed to communicate with the inner space 131, a strip vending unit 30 which is installed in the inner space 13, stores a plurality of strip kits 1 for testing urine, and provides the strip kits 1 to users through the strip outlet 19, a strip accommodation unit 42 which accommodates a case 5 configured to accommodate a strip 2 with urine entering the inner space 13 through the strip inlet 20, a urine analysis module 60 which analyzes urine by scanning the strip 2 accommodated in the case 5 put into the strip accommodation unit 42, a touch screen 22 through which unique identification information of the user is input, or a request for the strip vending unit 30 to discharge the strip kit 1 to the strip outlet 19 is input, a urine analysis control unit 96 which controls monitoring target data, which is acquired by the urine analysis module 60 analyzing the urine on the strip, to be transmitted to the outside or displayed on the touch screen 22, and a local communication unit 95 which transmits the monitoring target data to the outside.

In addition, the kiosk type urine analyzer 10 according to the first embodiment of the present invention includes a urine cup vending unit 65 in which a plurality of urine cups for storing urine are stacked in order to easily put the urine on the strip 2 and are discharged through a urine cup outlet 21 formed in the body 11 to correspond to the strip kit 1 by the urine analysis control unit 96, and a strip collection unit 75 which collects the strip kit 1 discharged from the strip accommodation unit 42 after urine analysis is completed in the urine analysis module 60.

The body 11 includes a main body 12 which is formed to be vertically long and has the inner space 13 that is open in one direction and in which the strip vending unit 30, the strip accommodation unit 42, the urine analysis module 60, the urine cup vending unit 65, and the strip collection unit 75 are accommodated in the inner space 13, and a door 18 which is rotatably connected to the main body 12 to open or close the inner space 13 of the main body 12 and in which the touch screen 22 is mounted, and the strip outlet 19, the strip inlet 20, and the urine cup outlet 21 are formed.

The body 11 further includes a discharge guide member 23 which is mounted to pass through the strip outlet 19 and of which an inner side is perforated such that a vertical thickness and a lateral width gradually decrease toward the strip vending unit 30, and an input guide member 24 which is mounted to pass through the strip inlet 20 and is perforated such that a vertical thickness and a lateral width gradually decrease toward the strip accommodation unit 42.

The strip outlet 19 and the strip inlet 20 are formed below the touch screen 22 which is mounted to be biased to one side at an upper portion of the door 18, and the urine cup outlet 21 is formed at the other side of a center of the door 18, but the present invention is not limited to the illustrated structure.

The touch screen 22 is an input/output device through which, when a command button, a keyboard, or the like is displayed such that a user can operate the screen, unique identification information of the user is input through a user touch, or a certain command for discharging the strip kit 1 from the strip vending unit 30 to the strip outlet 19 is input. The touch screen 22 may be exposed at the upper portion of the door 18, and when a urine analysis function is not performed, the touch screen 22 may display advertisement information or use information of the kiosk type urine analyzer 10 according to the first embodiment of the present invention.

Unique identification information input to the touch screen 22 may include a user's resident registration number, a number of a user terminal such as a mobile phone used by a user, a quick response (QR) code, a barcode, and the like. Unique identification information such as a resident registration number or a number of a user terminal may be input to the touch screen 22 through a user touch. When an application of a user terminal 6 is executed, unique identification information such as a QR code or a barcode may be displayed on a display unit of the user terminal 6, and the urine analyzer 10 may recognize and read the unique identification information such as the QR code or the barcode through a camera 25 provided above or around the touch screen.

The camera 25 may photograph a user while the user operates the touch screen 22, and a captured image may be stored in a memory 98 provided in the kiosk type urine analyzer 10 according to the first embodiment of the present invention together with the recognized unique identification information.

First and second vents 14 and 15 communicating with each other are formed to pass through an upper portion and a lower portion of one surface of the main body 12 such that the inner space communicates with an outer space. The kiosk type urine analyzer 10 according to the first embodiment of the present invention may further include an air cleaning unit 94 which is installed inside the main body 12 to purify outside air flowing into the second vent 15 formed on the upper of the main body and air in the inner space 13 and discharge the purified air to the first vent 14.

The strip vending unit 30 is installed in the inner space 13 and stores the plurality of strip kits 1 stored for testing urine to provide the strip kits 1 to users and is configured to discharge strip kits 1 one by one under the control of the urine analysis control unit 96.

Meanwhile, each strip kit 1 is packaged in a packaging bag 8 and provided to a user and includes the strip 2 and the case 5 into which the strip is inserted.

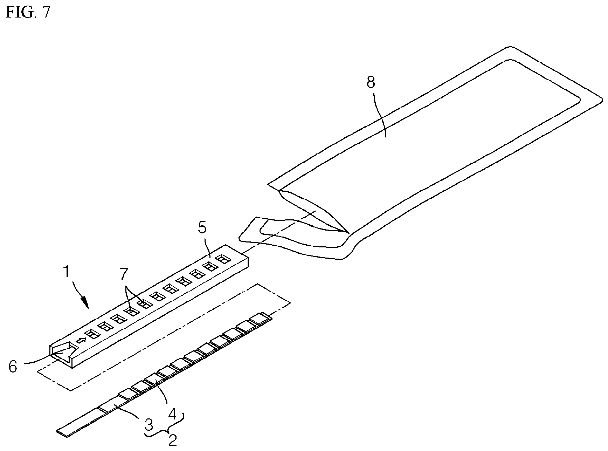

Referring to FIG. 7, the strip 2 includes a strip body 3 of which a length extends at a certain width, and a plurality of reagent pads 4 which are attached to one surface of the strip body 3 to be spaced a certain interval apart from each other to test items such as occult blood, bilirubin, urobilinogen, ketone bodies, protein, nitrites, glucose, acidity, specific gravity, leukocytes, and vitamin C.

A length of the case 5 extends at a certain width. The case 5 has a strip insertion groove 6 which corresponds to the strip body 3 or is recessed from one end toward the other end of the case 5 in a length direction to have a large width for the strip 2 to be inserted therein, and a plurality of first guide holes 7 which are formed to face the reagent pads 4 of the strip 2 which each pass through the strip insertion groove 6 and are inserted into the strip insertion groove 6.

While inserted into the case 5, the strip 2 is packaged in the packaging bag 8 to partially protrude therefrom. The strip 2 may be withdrawn from the case 5 to collect urine. After urine is collected, the strip 2 is inserted again into the case 5 and put into the strip accommodation unit 42.

The strip vending unit 30 includes a strip storage chamber 31, a strip pressing part 36, a withdrawal roller 39, and a withdrawal driving motor 40.

The strip storage chamber 31 has a rectangular pole shape vertically extending at a constant width and has an accommodation space 32 in which the plurality of strip kits 1 are stacked. The strip storage chamber 31 has an outlet 33 formed in a lower portion of a side surface thereof facing the door 18, which closes the inner space 13 of the main body 12, to communicate with the accommodation space 32 and the strip outlet 19, and a first roller entry hole 34 formed to pass through a lower end portion thereof facing downward and communicate with the accommodation space 32.

The strip pressing part 36 is installed between an upper end of the strip storage chamber 31 and the strip kit 1 disposed at an uppermost side among the plurality of strip kits 1 vertically stacked and presses the plurality of strip kits 1 downward. The strip pressing part 36 may include an elastic spring 37 which has an upper portion fixed to the upper end of the strip storage chamber 31 and of which a length is stretchable and contractible, and a plate-shaped pressure plate 38 mounted on a lower end of the elastic spring.

However, unlike that shown in the drawing, as the strip pressing part 36, a heavy object, which has a certain weight and is seated on an upper surface of the strip kit 1 disposed at the uppermost side among the plurality of vertically stacked strip kits 1 to press the plurality of strip kits 1 downward, may be applied.

One side of the withdrawal roller 39 enters the first roller entry hole 34 to support the strip kit 1 disposed at a lowermost side among the plurality of vertically stacked strip kits 1.

In order for the strip kit 1 supported by the withdrawal roller 39 to be discharged to the strip outlet 19 when the withdrawal roller 39 rotates, the withdrawal driving motor 40 is fixed to one side of the main body 12 such that a length of a withdrawal driving shaft 41, which is mounted to pass through a central portion of the withdrawal roller 39, extends in a direction perpendicular to a direction in which the outlet 33 is perforated.

When a command for withdrawing the strip kit 1 is input to the touch screen 22, the urine analysis control unit 96 controls the driving of the withdrawal driving motor 40 so that the withdrawal roller 39 is rotated and the strip kit 1 is discharged to the outside through the outlet 33 and the strip outlet 19.

The strip accommodation unit 42 is installed in the inner space 13 of the main body 12 and accommodates the strip kit 1 with urine. The strip accommodation unit 42 includes a strip holder 43, an accommodation roller 48, an accommodation driving motor 49, and an input detection sensor 51.

The strip holder 43 has an accommodation hole 44 of which one side communicates with the strip inlet 20 for the strip kit 1 to be accommodated therein, a light passing hole 45 which passes through an upper end portion thereof to communicate with the accommodation hole 44 such that light is radiated onto the plurality of reagent pads 4 of the strip 2 from the urine analysis module 60 positioned above or light reflected from the reagent pads 4 passes therethrough and which extends in a length direction in the form of a long hole, and a second roller entry hole 46 formed to pass through a lower end portion thereof to communicate with the accommodation hole 44. The strip holder 43 may partially close an open side of the inner space 13 of the main body 12, and one end thereof may be fixed to a front wall 16 in which a through-hole 17 communicating with the strip inlet 20 is formed. Alternatively, unlike that shown in the drawing, a structure in which the body 11 of the present invention is not provided with the front wall 16 and the strip holder 43 is mounted directly on the door 18 may be applied.

One side of the accommodation roller 48 enters the second roller entry hole 46 to come into contact with a lower surface of the strip kit 1 of which one side partially enters the accommodation hole.

The accommodation driving motor 49 rotates the accommodation roller 48 such that the strip kit 1 in contact with the accommodation roller 48 is moved into the main body 12 in a direction away from the strip inlet 20. The accommodation driving motor 49 is mounted on a lower surface of the strip holder 43 such that a length of an accommodation driving shaft 50, which is mounted to pass through a central portion of the accommodation roller 48, extends in a direction perpendicular to a direction in which the strip inlet 20 is perforated.

The input detection sensor 51 detects that the strip kit 1 has been inserted into the accommodation hole 44 and transmits a detection signal to the urine analysis control unit 96.

When a signal is transmitted from the input detection sensor 51, the urine analysis control unit 96 controls the driving of the accommodation driving motor 49 such that the accommodation roller 48 is primarily rotated in a direction opposite to a direction in which the withdrawal roller 39 is rotated. As the input detection sensor 51, a load sensor, which is mounted on an inner bottom surface of the strip holder 43 to detect the strip kit 1, may be applied, and a photometer, which detects brightness of light in the accommodation hole 44, may also be applied. That is, when a signal indicating a load of the strip kit 1 is transmitted, or a signal indicating a value less than or equal to set luminous intensity is transmitted, the urine analysis control unit 96 determines that the strip kit 1 has entered the accommodation hole 44 and drives the withdrawal driving motor 40.

The strip accommodation unit 42 may further include an accommodation completion detection sensor 52 for detecting that the strip kit 1 has been moved to the other side of the accommodation hole 44 by primary rotation of the accommodation roller 48. The accommodation completion detection sensor 52 may be positioned to close the other open end of the accommodation hole 44 and may be mounted on an end of a first plunger 77 of a first solenoid part 76 to be described below and moved together with the first plunger 77 to open the other side of the accommodation hole 44. As the accommodation completion detection sensor 52, a limit switch, which is operated by being pressed by the strip kit 1 moved by the primary rotation of the accommodation roller 48, may be applied.

In a state in which the other side of the accommodation hole 44 is closed by the accommodation completion detection sensor 52, when the accommodation completion detection sensor 52 is operated by being pressed by the strip kit 1 moved into the main body 12 by the first primary rotation of the withdrawal roller 39, the urine analysis control unit 96 operates the urine analysis module 60.

The urine analysis module 60 scans the strip 2 of the strip kit 1 put into the strip accommodation unit 42 and analyzes a urine component to perform a function of checking a health status of a user. As a method of checking a health status of a user through urine, there is a method of examining physical properties such as urine color/turbidity, a method of using semiquantitative chemical detection using a strip, a method of using a result of examining a component through a microscope, or the like. A method of checking a health status of a user through the urine analysis module 60 according to the present invention is not limited, but a method through a photodetection-based interface using the strip 2 is preferable.

For example, the urine analysis module 60 may include a plurality of light-emitting elements 61 which radiate light onto each reagent pad 4 of the strip 2 accommodated in the strip holder 43, a plurality of photosensors 64 which detect a color of the reagent pad 4 through light radiated from the light-emitting element 61 and reflected from the reagent pad 4, and a data processor (not shown) which generates a urine analysis result through a change in detected color according to an analysis standard stored in advance.

The urine analysis module 60 may further include an analysis guide member 62 having a plurality of second guide holes 63 formed to communicate with the plurality of first guide holes 7 to guide an optical path of each of the light-emitting elements 61

In the kiosk type urine analyzer 10 according to one embodiment of the present invention, the urine analysis control unit 96 may be applied to perform a function of the data processor. When urine analysis is completed, the strip kit 1 on which the urine analysis is completed is discharged to the strip collection unit 75, which will be described below.

Meanwhile, the urine cup outlet 21 is formed to be recessed from a front surface of the door 18, protrudes from a rear surface of the door 18, and is formed with an open upper side. The urine cup vending unit 65 is mounted on the door 18 to be positioned above the urine cup outlet 21 or is accommodated in the inner space 13 of the main body 12.

Although not specifically shown, the urine cup vending unit 65 may include a cup accommodation part vertically perforated above the urine cup outlet 21 to accommodate a plurality of cups, a pair of rotation levers which are disposed symmetrically with each other at a lower end of the cup accommodating part to grip a cup disposed at a lowermost side among a plurality of vertically stacked cups and are spread to move away from each other such that the gripped cup drops downward, a grip holding spring which protrudes toward the cup to elastically support one ends of the pair of rotation levers facing each other such that the pair of rotation levers keep gripping the cup, a push button which is open laterally to accommodate the one ends of the pair of rotation levers and the grip holding spring and which has a pressing groove of which a width gradually decreases toward a recessed end thereof, and an actuator 66 of which a length is stretchable or contractible under the control of the urine analysis control unit 96 to press or release the push button.

Briefly describing the operation of the urine cup vending unit 65 having such a structure, when the length of the actuator is stretched and the push button is pressed and moved, the pair of rotation levers are rotated such that the one ends of the pair of rotation levers further enter the pressing groove, of which the width is gradually decreased, to approach each other, and the other ends of the pair of rotation levers gripping the cup are rotated away from the cup. When the length of the actuator is contracted and a pressing force applied to the push button is released, the pair of rotation levers are rotated such that the one ends of the pair of rotation levers are moved away from each other by the grip holding spring, the push button is restored to the initial position thereof, and the other ends of the pair of rotation levers approach each other to grip the cup together.

The present invention is not limited to the structure described above as long as the urine cup vending unit 65 has a structure capable of discharging the urine cup through the urine cup outlet 21 at the same time when the strip kit 1 is withdrawn through the strip outlet 19 under the control of the urine analysis control unit 96.

Meanwhile, the strip collection unit 75 includes a first solenoid part 76 which moves the accommodation completion detection sensor 52 to open or close the other end of the accommodation hole 44 open to the other side of the strip holder 43 in a length direction, a waste strip collection chamber 82 having an opening 83 formed in an upper end portion thereof for the strip kit 1 discharged from the strip holder 43 due to the other end of the accommodation hole 44 being opened to be stored therein, an opening/closing door 84 which opens or closes the opening 83 of the waste strip collection chamber 82, and a second solenoid part 88 which slides the opening/closing door 84 to open or close the opening 83.

The first solenoid part 76 includes a first coil housing 79 mounted on a side portion of a support bracket 81 mounted to protrude from one side of the main body 12 such that an open portion thereof faces upward, a first plunger 77 which is mounted to enter or retreat from the first coil housing 79 and on an end of which protruding from the first coil housing 79 the accommodation completion detection sensor 52 is mounted, a first electromagnetic coil 78 accommodated in the first coil housing 79 and formed by winding a conductor in a cylindrical shape around the first plunger 77, and a first return spring 80 which supports the first plunger 77 in a hollow portion formed in the first electromagnetic coil 78.

When the first electromagnetic coil 78 is energized, the first electromagnetic coil 78 generates a magnetic field by which the first plunger 77 may be pulled into the first coil housing 79. When the first electromagnetic coil 78 is de-energized, the first return spring 80 elastically supports the first plunger 77 such that the accommodation completion detection sensor 52 closes the other end of the accommodation hole 44.

The waste strip collection chamber 82 is formed in a rectangular column shape, a plurality of through-holes are formed in each side at a lower portion thereof, and a box 92 in which a solid body 93 for emitting chlorine dioxide (ClO₂) gas is accommodated is stored therein.

The opening/closing door 84 includes a plate-shaped opening/closing body 85 formed to cover the opening 83, and a drop guide part 86 which extends upward from one end of the opening/closing body 85 facing the strip accommodation unit 42 and is formed with an upper portion thereof bent toward the strip accommodation unit 42.

Although not specifically shown, the second solenoid part 88 includes a second coil housing, a second plunger 89, a second electromagnetic coil, and a second return spring.

The second coil housing is supported on an upper end of the waste strip collection chamber 82 or is supported on a second support bracket (not shown) mounted to protrude from one side of the main body 12 and is mounted such that an open side thereof faces the door 18 that closes the inner space 13 of the main body 12.

The second plunger 89 is mounted to enter or retract from the second coil housing and is parallel to an upper surface of the waste strip collection chamber 82, and one end portion thereof protruding from the second coil housing is coupled to an upper surface of the other end portion of the opening/closing body 85. The second electromagnetic coil is accommodated in the second coil housing and is formed by winding a conductor in a cylindrical shape around the second plunger 89. The second return spring elastically supports the other end portion of the second plunger in a hollow portion formed in the second electromagnetic coil.

When the second electromagnetic coil is energized, the second electromagnetic coil generates a magnetic field by which the second plunger 89 may be pulled into the second coil housing to open the opening 83. When the second electromagnetic coil is de-energized, the second return spring elastically supports the second plunger 89 to close the opening 83.

The strip collection unit 75 includes a discharge guide tray 90 which extends downward from the other end of the strip holder 43 toward the opening 83 of the waste strip collection chamber 82 and is open in a length direction and an upward direction, and a pair of separation prevention ribs 91 which are spaced apart from each other with the opening 83 interposed therebetween, of which a length extends in a direction in which the strip kit 1 is discharged, and which are mounted on the upper end of the waste strip collection chamber 82.

When urine analysis is completed in the urine analysis module 60, the urine analysis control unit 96 applies electricity to the first and second electromagnetic coils to open the other end of the accommodation hole 44 and the opening 83 of the waste strip collection chamber 82 and drives the accommodation driving motor 49 such that the accommodation roller 48 is secondarily rotated in the same direction as the primary rotation direction.

As the accommodation roller 48 is secondarily rotated, the strip kit 1 on which urine analysis is completed is discharged from the strip accommodation unit 42 and stored in the waste strip collection chamber 82.

When the strip kit 1 is collected in the waste strip collection chamber 82, the urine analysis control unit 96 releases the application of electricity to the first and second electromagnetic coils to close the other end of the accommodation hole 44 and the opening 83 of the waste strip collection chamber 82.

When a certain period of time has elapsed after a signal is transmitted from the accommodation completion detection sensor 52, the urine analysis control unit 96 may be applied to apply electricity to the first and second electronic coils and drive the accommodation driving motor 49. In addition, when a certain period of time has elapsed after electricity is applied to the first and second electronic coils, the urine analysis control unit 96 may be applied to release the application of electricity to the first and second electromagnetic coils and stop the accommodation driving motor 49.

Meanwhile, although not specifically shown, the air cleaning unit 94 may have a structure of a general air cleaner purifier including a housing which includes an inlet port perforated to communicate with the inner space of the main body 12 and an outlet port perforated to communicate with the first vent 14 and has an inner space, a suction fan installed in the housing to suction air in the inner space of the main body 12 such that air in an outer space flows into the inner space of the main body 12 through the second vent 15, a blower which includes a driving motor for driving the suction fan, and a filter which filters contaminants from the air flowing into the housing.

The filter may include various types of filters such as a prefilter, a high efficiency particulate air filter (HEPA filter), and a deodorization filter.

The air cleaning unit 94 may further include a plurality of ultraviolet lamps which radiate ultraviolet light having at least two different wavelengths among short-wavelength ultraviolet (UV-C) light, medium-wavelength ultraviolet (UV-B) light, and long-wavelength ultraviolet (UV-A) light to sterilize air flowing into the housing.

The air cleaning unit 94 may further include a drying heater which includes a plurality of heating wires and the like at a position between the inlet port and the filter in the housing to remove moisture from air suctioned into the inlet port and then supply air to the filter, and a humidity sensor which measures humidity of air flowing into the housing and transmits a measured signal to the urine analysis control unit. When a humidity value transmitted from the humidity sensor is greater than or equal to a set value, the urine analysis control unit 96 may control and drive the drying heater.

The local communication unit 95 may include a short-distance communication module which performs short-distance communication with a user terminal such as a smartphone, and a terminal communication module which may communicate with the user terminal through a web or an application using a general wireless communication method.

The memory 98 may store monitoring target data which is a result of the urine analysis module 60 analyzing urine on the strip.

The urine analysis control unit 96 may transmit the monitoring target data stored in the memory 98, that is, a urine analysis result, to a user terminal such as a smartphone through the local communication unit 95.

The kiosk type urine analyzer 10 according to the first embodiment of the present invention may further include a second ultraviolet sterilizer (not shown) mounted at one side of the strip holder to sterilize the inside of the strip holder 43. The second ultraviolet sterilizer may further include a general ultraviolet lamp which emits light to sterilize the accommodation hole 44 in a state in which the accommodation hole 44 of the strip holder 43 is empty. The ultraviolet lamp may be driven and controlled by the urine analysis control unit 96, and the urine analysis control unit 96 may be applied to control the accommodation roller 48 to be driven for a certain time when the accommodation roller 48 is secondarily rotated.

Since, after a urine test is performed, a test result can be provided to a user terminal such as a smartphone, and users can receive the test result after the urine test if necessary without visiting a hospital or purchasing a separate urine analyzer, the kiosk type urine analyzer 10 according to the first embodiment of the present invention having such a structure has an advantage in that use efficiency thereof is high.

In the kiosk type urine analyzer 10 according to the first embodiment of the present invention, when the strip 2 with urine partially enters the main body 12, the strip 2 can be moved vertically downward from the urine analysis module 60 to be automatically accommodated, and after urine analysis is completed, the strip 2 with urine can be automatically collected in the waste strip collection chamber 82 so that strip treatment can be easy and management efficiency can be improved.

In addition, in the kiosk type urine analyzer 10 according to the first embodiment of the present invention, since the plurality of first guide holes 7 formed in the case 5 for accommodating the strip 2 in the strip holder 43 are positioned vertically below the light-emitting elements and the photosensors with the strip 2 interposed therebetween, urine analysis efficiency can be improved.

In the kiosk type urine analyzer 10 according to the first embodiment of the present invention, unlike that described above, a structure in which the strip 2 is packaged in the packaging bag 8 without the case 5 and only the strip 2 is put into the strip holder 43 may be applied.

Meanwhile, a health monitoring system using a kiosk type urine analyzer according to a first embodiment of the present invention includes at least one kiosk type urine analyzer 10 capable of providing a strip for a urine test to a user and transmitting monitoring target data, which is acquired by analyzing urine on the collected strip, to the outside, a database server 120 which stores each of patient group urine analysis data acquired by analyzing urine of a patient group requiring treatment for a chronic disease such as diabetes or nephritis, control group urine analysis data acquired by analyzing urine of a control group without a chronic disease, and past urine analysis data of the user, and a health monitoring server 100 which monitors a health status of the user with respect to a chronic disease through a deep learning algorithm based on data stored in the database server 120 and transmits a monitoring result to a user terminal 6 capable of performing wireless communication with the kiosk type urine analyzer 10.

The database server 120 includes a patient group database 121 which stores the patient group urine analysis data acquired by analyzing the urine of the patient group requiring treatment for a chronic disease such as diabetes or nephritis, a control group database 123 which stores the control group urine analysis data acquired by analyzing the urine of the control group without a chronic disease, and a user database 125 which stores the past urine analysis data of the user.

The patient group database 121 stores urine analysis data about items such as occult blood, bilirubin, urobilinogen, ketone bodies, protein, nitrites, glucose, acidity, specific gravity, leukocytes, and vitamin C of a patient with a chronic disease, and data about a residential area, an age, a sex, a height, a weight, or a blood type thereof together.

In addition, the control group database 123 stores urine analysis data about items such as occult blood, bilirubin, urobilinogen, ketone bodies, protein, nitrites, glucose, acidity, specific gravity, leukocytes, and vitamin C of ordinary people without a chronic disease, and data about a residential area, an age, a sex, a height, a weight, or a blood type thereof together.

The user database stores not only urine analysis data about items such as occult blood, bilirubin, urobilinogen, ketone bodies, protein, nitrites, glucose, acidity, specific gravity, leukocytes, and vitamin C of the user but also data about a residential area, an age, a sex, a height, a weight, and a blood type thereof together.

The health monitoring server 100 includes an application programming interface (API) 101 which receives a monitoring target data from the kiosk type urine analyzer 10 through a wireless network or transfers a health monitoring result to the kiosk type urine analyzer 10 and receives data from the database server 120, and a deep learning monitoring model 150 which selects data information required for learning from patient group urine analysis data acquired by analyzing urine of a patient with a chronic disease and control group urine analysis data acquired by analyzing urine of healthy ordinary people without a chronic disease and constructs labeled learning data, and a deep learning health monitoring model 105 which, when monitoring target data is input, derives a health monitoring result of a user with respect to a chronic disease such as diabetes or nephritis through the deep learning algorithm based on the learning data.

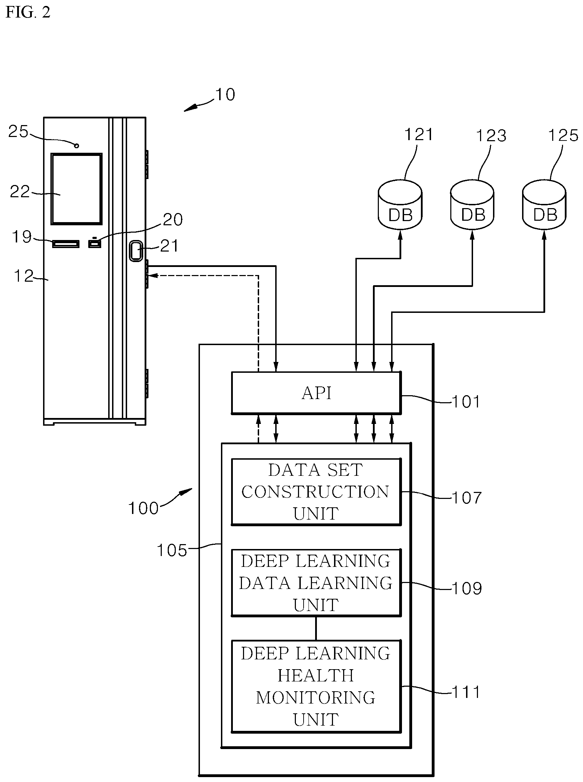

Referring to FIG. 2, the deep learning health monitoring model 105 includes a data set construction unit which selects the data information required for learning from the patient group urine analysis data acquired by analyzing the urine of the patient with a chronic disease and the control group urine analysis data acquired by analyzing the urine of the healthy ordinary people without a chronic disease and constructs a patient group data set and an ordinary people data set for learning, a deep learning data learning unit 109 which learns the patient group data set and the ordinary person data set using a deep learning engine among artificial intelligence (AI) algorithms and derives an inference model, and a deep learning health monitoring unit 111 which monitors a health status of a user with respect to a chronic disease through the inference model when urine analysis data of the user, that is, monitoring target data and data about a residential area, an age, a sex, a height, a weight, and a blood type of the user, are input.

When personal information of a user and urine analysis data about occult blood, bilirubin, urobilinogen, ketone bodies, protein, nitrites, glucose, acidity, specific gravity, leukocytes, and vitamin C of monitoring target data are input, the deep learning health monitoring unit 111 may monitor a health status of the user through an inference model.

Although not shown, the health monitoring server 100 may include a central control unit which is connected to the API 101 to collect monitoring target data and information about the kiosk type urine analyzer 10 received through a wireless network, controls a result monitored by the deep learning health monitoring unit 111 to be transmitted to the kiosk type urine analyzer 10 and the user database 125, and controls the deep learning health monitoring model 105 to monitor the monitoring target data when the monitoring target data is transmitted.

In the health monitoring system according to the first embodiment of the present invention, there is an advantage in that it is possible to monitor a health status of a user with respect to a chronic disease by comprehensively analyzing monitoring target data acquired by the kiosk type urine analyzer 10 through a deep learning model based on urine analysis data of the user accumulated from the past and urine analysis data of patient and control groups.

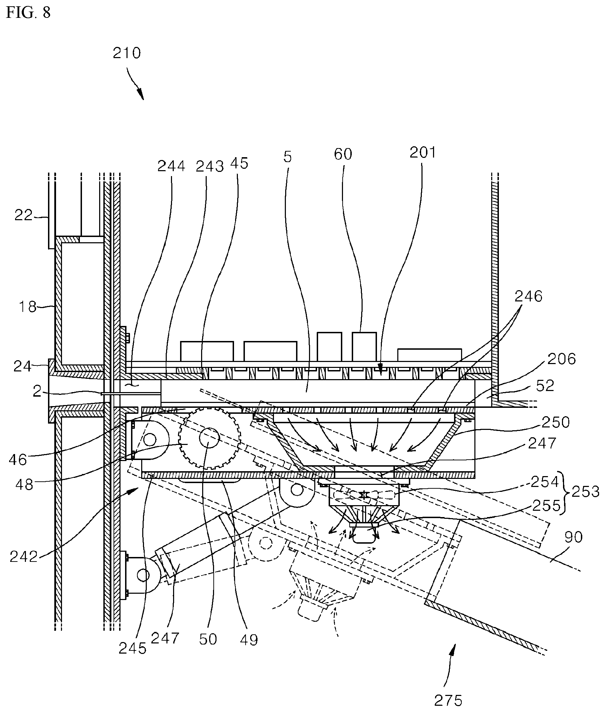

Meanwhile, FIG. 8 shows a kiosk type urine analyzer 210 according to a second embodiment of the present invention. Components having the same functions as components shown above in the drawings are denoted by the same reference numerals. The kiosk type urine analyzer 210 according to the second embodiment of the present invention has the same structure as the first embodiment of the present invention except for a strip accommodation unit, a strip collection unit 275, and a strip kit 201.

The strip accommodation unit 242 includes an upper strip holder 243, a lower strip holder 245, a rotation operation cylinder 247, an accommodation roller 48, and an accommodation driving motor 49.

The upper strip holder 243 includes an accommodation hole 244 of which one side communicates with a strip inlet 20 to accommodate a strip 2 in a length direction thereof, and the other end is closed by an accommodation completion detection sensor 52 and opens downward, and a light passing hole 45 which is formed to pass through an upper end portion thereof to communicate with the accommodation hole 244 such that light is radiated onto a plurality of reagent pads 4 of the strip 2 from a urine analysis module 60 positioned above or light reflected from the reagent pads 4 passes therethrough.

The lower strip holder 245 closes a lower side of the accommodation hole 244, supports the strip kit 201 accommodated in the accommodation hole 244, and is mounted on a main body 12 or a door 18 to be rotated and inclined downward such that the strip 2 analyzed by the urine analysis module 60 is discharged downward. The lower strip holder 245 has a second roller entry hole 46 formed to pass through an upper end portion thereof and communicate with the accommodation hole 244.

One side of the rotation operation cylinder 247 is connected to the main body 12 or the door 18, the other side thereof is connected to a lower portion of the lower strip holder 245, and a length thereof is stretched and contracted by a urine analysis control unit 96 to rotate the lower strip holder 245.

The accommodation roller 48 is rotatably mounted in the lower strip holder 245 such that one side thereof enters the second roller entry hole 46 to support the strip kit 201 which partially enters the accommodation hole 244. The accommodation driving motor 49 is controlled and driven by the urine analysis control unit 96 to rotate the accommodation roller 48 such that the strip kit 201 supported by accommodation rollers 48 is moved into a main body 12 in a direction away from a strip inlet 20.

The strip collection unit 275 has the same structure as the strip collection unit 75 according to the first embodiment of the present invention except that a first solenoid part 76 is not provided in a structure of the strip collection unit 75 according to the first embodiment of the present invention, and an upper end of the discharge guide tray 90 is disposed in contact with the lower strip holder 245 rotated downward.

The lower strip holder 245 has an inner space having a certain height formed therein, a plurality of first vent holes 246 formed at an upper end portion thereof, and one second vent hole 247 formed at a lower end portion thereof. The kiosk type urine analyzer 210 according to the second embodiment of the present invention may further include a duct part 250 and a blowing fan 253.

The duct part 250 is accommodated in an inner space of the lower strip holder 245, extends downward along a circumference formed by the plurality of first vent holes 246 to connect the first vent holes 246 and the second vent hole 247, and is formed in a shape having a wide upper portion and a narrow lower portion.

The blowing fan 253 is mounted on a lower end of the lower strip holder 345 to be connected to a lower end of the duct part 250 and includes a blade 254 of which a rotational direction is changeable such that air of the first vent hole 246 is moved downward, or air below the lower strip holder 245 flows into the first vent hole 246, and a fan motor 255 for rotating the blade.

When the input of the strip kit 201 is detected by an input detection sensor 51, the urine analysis control unit 96 rotates the accommodation roller 48, and when it is determined that the accommodation of the strip kit 201 is completed by the accommodation completion detection sensor 52, the urine analysis control unit 96 drives the urine analysis module 60 to drive the blowing fan 253 such that air in the lower strip holder 245 is suctioned downward.

When a set time has elapsed or an analysis completion signal is transmitted from the urine analysis module 60, the urine analysis control unit 96 decreases a length of the rotation operation cylinder 247 to rotate the lower strip holder 245 to be inclined downward and controls and drives the blowing fan 253 such that air is blown toward the strip kit 201 through the first vent hole 246.

The strip kit 201 includes a strip 2, a case 5, and a contact guide part 206 which covers a lower surface of the case 5 and is formed of a contractible material such as rubber.

A frictional force between the accommodation roller 48 and the strip kit 201 may be increased by the contact guide part 206 so that the sliding movement of the strip kit 1 by the rotation of the accommodation roller 48 may be facilitated. When air in the lower strip holder 245 is suctioned toward the blowing fan 253, the strip kit 201 may be in close contact with the lower strip holder 245 to be stably fixed while the urine analysis module 60 analyzes urine.

In the kiosk type urine analyzer 210 according to the second embodiment of the present invention, while the urine analysis module 60 analyzes urine, air may be suctioned downward through the first vent hole 246 so that the strip kit 201 may be stably fixed to the lower strip holder 245. After urine analysis is completed, the lower strip holder 245 is rotated downward and air is blown toward the strip kit 201 through the first vent hole 246, thereby improving efficiency of discharging the strip kit 201 to the strip collection unit 75.

In a health monitoring system of the present invention, there is an advantage in that it is possible to monitor a health status of a user with respect to a chronic disease by comprehensively analyzing monitoring target data acquired by a kiosk type urine analyzer through a deep learning model based on urine analysis data of a user accumulated from the past and urine analysis data of patient and control groups.

Since, after a urine test is performed, a test result can be provided to a user terminal such as a smartphone, and users can receive the test result after the urine test if necessary without visiting a hospital or purchasing a separate urine analyzer, a kiosk type urine analyzer of the present invention has an advantage in that use efficiency thereof is high.

In a kiosk type urine analyzer of the present invention, when a strip with urine partially enters a body, the strip can be moved vertically downward from a urine analysis module to be automatically accommodated, and after urine analysis is completed, the strip with urine can be automatically collected in a waste strip collection chamber so that strip treatment can be easy and management efficiency can be improved.

In addition, in a kiosk type urine analyzer of the present invention, since a plurality of first guide holes formed in a case for accommodating a strip in a strip holder are positioned vertically below light-emitting elements and photosensors with the strip interposed therebetween, urine analysis efficiency can be improved.

The present invention has been described with reference to the embodiments shown in the drawings, but these are merely examples, and those skilled in the art will appreciate that various modifications, additions and substitutions are possible. Accordingly, the true protection scope of the present invention should be defined only by the appended claims.

## Claims

1. A kiosk type urine analyzer comprising:
a body having an inner space and a strip outlet and a strip inlet which are formed to communicate with the inner space;
a strip vending unit installed in the inner space and configured to store a plurality of strips for testing urine and provide the strips to users through the strip outlet;
a strip accommodation unit configured to accommodate a strip with urine which enters the inner space through the strip inlet;
a urine analysis module configured to scan the strip put in the strip accommodation unit to analyze the urine;
a touch screen through which unique identification information of a user is input or a request for the strip vending unit to discharge the strip to the strip outlet is input;
a urine analysis control unit configured to control monitoring target data, which is acquired by the urine analysis module analyzing the urine on the strip, to be transmitted to an external device or displayed on the touch screen; and
a local communication unit configured to transmit the monitoring target data to the external device.

2. The kiosk type urine analyzer of claim 1, wherein the strip vending unit includes:
a strip accommodation chamber having an accommodating space which vertically extends therein for the plurality of strips to be stacked therein, an outlet formed in a lower portion of a side surface thereof facing the door to communicate with the accommodation space and the strip outlet, and a first roller entry hole formed to pass through a lower end portion thereof facing downward and communicate with the accommodation space;
a withdrawal roller of which one side enters the first roller entry hole to support the strip disposed at a lowermost side among the plurality of vertically stacked strips;
a strip pressing part configured to press the strip disposed at an uppermost side among the plurality of strips downward; and
a withdrawal driving motor controlled and driven by the urine analysis control unit to rotate the withdrawal roller such that the strip supported by the withdrawal roller is discharged to the strip outlet.

3. The kiosk type urine analyzer of claim 1, wherein the strip accommodation unit includes:
a strip holder having an accommodation hole of which one side communicates with the strip inlet to accommodate the strip, a light passing hole which passes through an upper end portion thereof to communicate with the accommodation hole such that light is radiated onto a plurality of reagent pads of the strip from the urine analysis module positioned above or light reflected from the reagent pads passes therethrough, and a second roller entry hole formed in a lower end portion thereof to communicate with the accommodation hole;
an accommodation roller of which one side enters the second roller entry hole to support the strip which partially enters the accommodation hole; and
an accommodation driving motor controlled and driven by the urine analysis control unit to primarily rotate the accommodation roller such that the strip supported by the accommodation roller is moved into the body in a direction away from the strip inlet.

4. The kiosk type urine analyzer of claim 3, wherein:
the accommodation hole is formed by opening the other end of the strip holder in a length direction; and
the kiosk type urine analyzer further includes:
a first solenoid part including a first plunger which is movable to close the other end of the accommodation hole until the urine analysis module completes the analysis of the urine on the strip accommodated in the strip holder and open the other end of the accommodation hole when the analysis of the urine is completed;
a waste strip collection chamber having a waste strip collection hole formed in an upper portion thereof such that, in a state in which the other end of the accommodation hole is opened, as the accommodation roller is secondarily rotated in the same direction as a direction in which the accommodation roller is primarily rotated, the strip discharged from the strip holder to the inner space of the body drops to be stored therein;
a collection hole opening/closing door slidably mounted on an upper portion of the waste strip collection chamber to open or close the waste strip collection hole; and
a second solenoid part including a second plunger which is movable together with the collection hole opening/closing door such that the waste strip collection hole is also opened or closed when the other end of the accommodation hole is opened or closed.

5. The kiosk type urine analyzer of claim 1, wherein:
the strip is provided from the strip vending unit after being inserted into a case such that a portion thereof protrudes from the case, is withdrawn from the case to collect the urine, and is reinserted into the case and put into the strip accommodation unit after the urine is collected; and
the case has a length that extends at a certain width and includes a strip insertion groove which is recessed from one end toward the other end thereof in a length direction for the strip to be inserted therein, and a plurality of first guide holes which are formed to face reagent pads of the strip which each pass through the strip insertion groove from an upper surface thereof.

6. The kiosk type urine analyzer of claim 1, wherein:
a first vent and a second vent are formed to pass through an upper portion and a lower portion of one surface of the body such that the inner space communicates with an outer space; and
the kiosk type urine analyzer further includes an air cleaning unit which is installed in the inner space to purify outside air flowing into the second vent formed on the upper portion of the body and air in the inner space.

7. The kiosk type urine analyzer of claim 1, wherein the strip accommodation unit includes:
an upper strip holder including an accommodation hole of which one side communicates with the strip inlet to accommodate the strip in a length direction thereof and the other side is closed and opens downward, and a light passing hole which is formed to pass through an upper end portion thereof to communicate with the accommodation hole such that light is radiated onto a plurality of reagent pads of the strip from the urine analysis module positioned above or light reflected from the reagent pads passes therethrough;
a lower strip holder which closes a lower side of the accommodation hole, supports the strip accommodated in the accommodation hole, is rotated and inclined downward such that the strip analyzed by the urine analysis module is discharged downward, and has a plurality of vent holes and a second roller entry hole which are formed to pass through an upper end portion thereof and communicate with the accommodation hole;
a rotation operation cylinder of which one side is connected to the body, the other side is connected to a lower portion of the lower strip holder, and a length is stretched and contracted to rotate the lower strip holder;
an accommodation roller of which one side enters the second roller entry hole to support the strip partially entering the accommodation hole and which is rotatably mounted on the lower strip holder;
an accommodation driving motor controlled and driven by the urine analysis control unit to rotate the accommodation roller such that the strip supported by the accommodation roller is moved into the body in a direction away from the strip inlet.
a duct part extending downward along a circumference formed by the plurality of vent holes; and
a blowing fan which is mounted on a lower end of the duct part and includes a blade of which a rotational direction is changeable such that air below the lower strip holder flows into the vent hole.

8. A health monitoring system comprising:
a kiosk type urine analyzer configured to provide a strip for a urine test to a user and transmit monitoring target data acquired by analyzing urine on the collected strip to an external device;
a database server configured to store each of patient group urine analysis data acquired by analyzing urine of a patient group requiring treatment for a chronic disease such as diabetes or nephritis, control group urine analysis data acquired by analyzing urine of a control group without a chronic disease, and past urine analysis data of the user; and
a health monitoring server configured to, when the monitoring data transmitted from the kiosk type urine analyzer is input, monitor a health status of the user with respect to the chronic disease through a deep learning algorithm based on data stored in the database server and transmit a monitoring result to a user terminal configured to perform wireless communication with the kiosk type urine analyzer.

9. The health monitoring system of claim 8, wherein the kiosk type urine analyzer includes:
a body having an inner space and a strip outlet and a strip inlet which are formed to communicate with the inner space;
a strip vending unit installed in the inner space and configured to store a plurality of strips for testing urine and provide the strips to users through the strip outlet;
a strip accommodation unit configured to accommodate the strip with the urine which enters the inner space through the strip inlet;
a urine analysis module configured to scan the strip put in the strip accommodation unit to analyze the urine;
a touch screen through which unique identification information of the user is input or a request for the strip vending unit to discharge the strip to the strip outlet is input; and
a urine analysis control unit configured to control monitoring target data, which is acquired by the urine analysis module analyzing the urine on the strip, to be transmitted to the external device or displayed on the touch screen; and
a local communication unit configured to transmit the monitoring target data to the external device.
